(19) Europäisches Patentamt
European Patent Office
Office européen des brevets

(11) Publication number: **0 193 855**
**B1**

(12) # EUROPEAN PATENT SPECIFICATION

(45) Date of publication of patent specification: **18.07.90**

(21) Application number: **86102476.8**

(22) Date of filing: **26.02.86**

(51) Int. Cl.$^5$: **C 07 D 491/107,**
A 61 K 31/415 //
(C07D491/107, 235:00,
311:00)

(54) **Hydantoin derivatives, processes for preparing them and pharmaceutical compositions containing them.**

(30) Priority: **26.02.85 JP 35236/85**

(43) Date of publication of application:
**10.09.86 Bulletin 86/37**

(45) Publication of the grant of the patent:
**18.07.90 Bulletin 90/29**

(84) Designated Contracting States:
**AT BE CH DE FR GB IT LI NL SE**

(56) References cited:
**EP-A-0 034 063**
**EP-A-0 092 386**
**DE-A-3 128 606**

(73) Proprietor: **Eisai Co., Ltd.**
**6-10, Koishikawa 4-chome Bunkyo-ku**
**Tokyo 112 (JP)**

(72) Inventor: **Ueda, Kouichiro**
**3-10-5, Tsubakiyama**
**Hasuda-shi Saitama Prefecture (JP)**
Inventor: **Nomura, Keiichi**
**6-12-8, Izumi-cho**
**Houya-shi Tokyo (JP)**
Inventor: **Tanaka, Satoru**
**43-47, Izumi**
**Abiko-shi Chiba Prefecture (JP)**
Inventor: **Nakai, Noboru**
**2553-4, Ishisaka**
**Toyama-shi Toyama Prefecture (JP)**

(74) Representative: **Lehn, Werner, Dipl.-Ing. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 193 855 B1**

**Description**

Background of the Invention

1) Field of the Invention:

This invention relates to hydantoin derivatives and their pharmacologically acceptable salts, both having excellent pharmaceutical effects for treating and preventing a chronic condition of disease and a combined condition of disease accompanied by diabetes, their preparation process and pharmaceutical compositions containing same.

2) Description of the Prior Art:

A number of antidiabetic drugs such as those containing sulfonylurea, mesoxalates, guanidine derivatives have been put on the market to date. They are however all symptomatic therapeutic drugs for hyperglycemia and there is no fully-effective antidiabetic drug.

Especially, there is practically no therapeutic drugs for various chronic symptoms and complications accompanying diabetes, for example, diabetic cataracts, diabetic neuropathy and diabetic retionpathy. Under the circumstances, it may not be too much to say that there is absolutely no effective therapeutic method for such diseases. Particularly, there is practically no effective medicinal treatment method for cataracts due to phacoscotasmus.

With the foregoing in view, many studies have been carried out for many years with a view toward developing effective therapeutic drugs for such intractable diseases. However, the present inventors are practically unaware of any successful studies.

One of the objectives of such studies was to discover an aldose reductase inhibitor. In "Science", 182, 1146—8, (1973), J. H. Kinoshita et al reported a theory that in a patient suffering from diabetic cataracts, the activity of aldose reductase in the crystalline lens increases, sugars such as glucose and galactose which are introduced in the crystalline lens are reduced by the enzyme into polyols such as sorbitol and galactitol, and the resulting accumulation of these polyols serves as a principal caused for stroma lesions of the crystalline lens. Since then, discovery of aldose reductase inhibitors has been the subject of a great deal of work on the basis of the theory of J. H. Kinoshita et al.

DE—A—31 28 606 discloses hydantoin derivatives whose benzene ring is not substituted by an amino or nitro group. These compounds do not possess superior solubility as well as an equally good aldose reductase inhibitory activity as the compounds according to the invention.

EP—A—34 063 describes also hydantoin derivatives which do, however, not carry a mono-methyl group in 3-position of the chroman skeleton. Also these compounds do not possess such an exceptionally good aldose reductase inhibitory effect as the claimed compounds.

Summary of the Invention

The present inventors have carried out an investigation with a view toward finding out such an aldose reductase inhibitor. As a result, they have found compounds having extremely strong aldose reductase inhibitory activities, leading to completion of this invention.

In one aspect of this invention, the present invention provides a hydantoin derivative having the general formula (I):

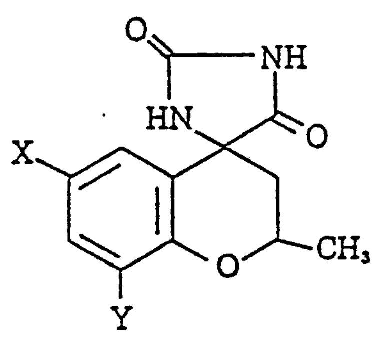

( I )

wherein X represents a halogen atom and Y represents an amino or nitro group or a pharmacologically acceptable salt thereof.

In another aspect of this invention, there is provided a process for the preparation of a compound having the general formula:

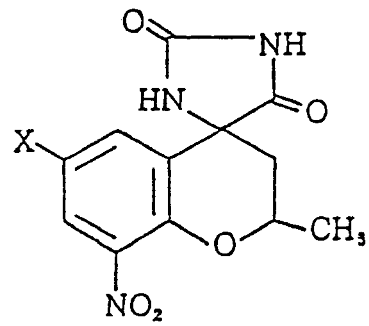

wherein X represents a halogen atom, which comprises nitrating a compound having the general formula:

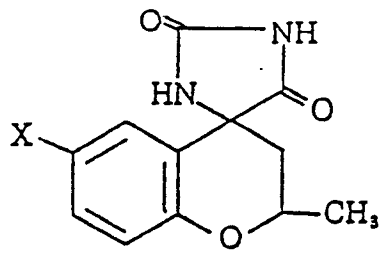

wherein X has the same meaning as defined above.

In a further aspect of this invention, there is provided a process for the preparation of a compound having the general formula:

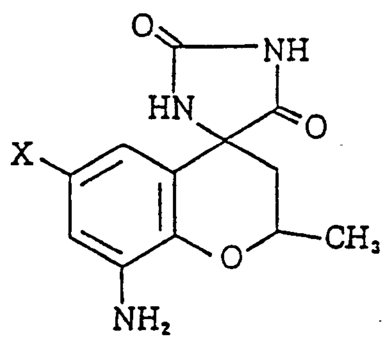

wherein X represents a halogen atom, which comprises reducing a compound having the general formula:

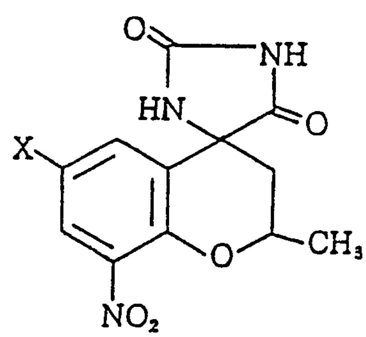

wherein X has the same meaning as defined above.

In a further aspect of this invention, there is also provided a treating and preventing composition for a chronic condition of disease and a combined condition of disease accompanied by diabetes, which comprises, as an essential component, a hydantoin derivative having the general formula (I) or a pharmacologically acceptable salt thereof.

The hydantoin derivatives of the general formula (I) and their pharmacologically acceptable salts are effective for the treatment and prevention of various chronic symptoms and complications accompanying diabetes.

In addition, the hydantoin derivatives of this invention have good water solubility. Therefore, these compounds are advantageous in making the preparation in the form of the water-soluble formulation, for example, dropping lotion in the eyes.

The above and other objects, features and advantages of this invention will become apparent from the following description and appended claims.

Detailed Description of the Invention and Preferred Embodiments

In the above-described general formula (I), the term "halogen atom" referred to in the definition for X means chlorine, bromine, iodine or fluorine atom specifically.

The term "pharmacologically acceptable salt" as used herein means specifically a cation salt such as sodium, potassium, calcium or magnesium salt.

Furthermore, each of the compounds of this invention includes various optical isomers because it contains 1 to 2 asymmetric carbon atoms owing to its structure. Needless to say, all of such optical isomers are embraced in the scope of the present invention.

A variety of processes may be contemplated to prepare each compound (I) of this invention. The following process may be mentioned as a representative process among such processes.

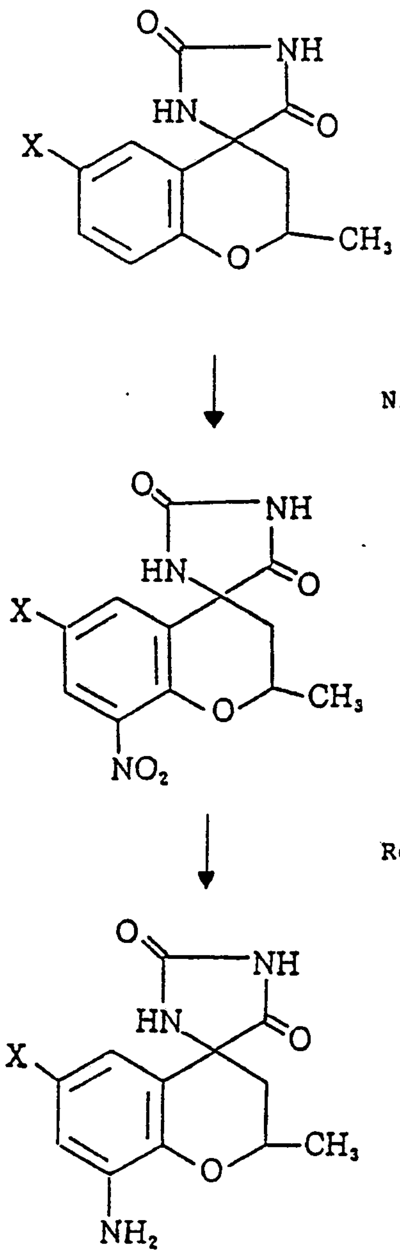

(II)

Nitration

(I')

(Wherein X has the same meaning as defined above.)

Reduction

(I")

(Wherein X has the same meaning as defined above.)

Namely, the compound having the general formula (I'') which is the desired material of the present invention can be obtained by nitrating the hydantoin derivative having the general formula (II) with conc. nitric acid in a usual manner to convert it into a compound having the general formula (I'), which is one of the desired materials of this invention, and then reducing the nitro group in a usual manner into an amino group. Preferable results may be obtained by effecting the reduction with a metal such as iron, zinc or tin and an acid such as hydrochloric acid or acetic acid.

Incidentally, the compound employed as a starting material and having the general formula (II) may be obtained by a process disclosed, for example, in JP—32793/88.

Namely, the compound having the general formula (II) may be obtained by condensing the following three components, i.e., (1) the compound having the general formula (III):

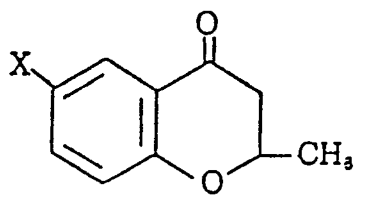

(Wherein X has the same meaning as defined above.) (III)

and (2) an alkali metal cyanide such as potassium cyanide or sodium cyanide and ammonium carbonate in a usual manner.

An Experiment will next be described in order to describe the effects of certain compounds of this invention in detail.

Experiment:

Aldose reductase inhibitory activity:

Aldose reductase was prepared by the method of Hayman et al [S. Hayman and J. H. Kinoshita, Journal of Biological Chemistry, 240, 877 (1965)], and the inhibition activity on aldose reductase was determined by the method of Gabbay et al [K. H. Gabbay and J. H. Kinoshita, Method in Enzymology, 41, 159 (1975)].

Results are summarized in Table 1, in which $ID_{50}$ indicates the concentration of 50% inhibition on aldose reductase.

The following compounds were employed for clinical tests, which will hereinafter be designated as Compounds A—C respectively.

Compound A: 6-Fluoro-8-nitro-2-methyl-spiro-[chroman-4,4'-imidazolidine]-2',5'-dione.
Compound B: 6-Fluoro-8-amino-2-methyl-spiro-[chroman-4,4'-imidazolidine]-2',5'-dione.
Compound C: 6-Chloro-8-amino-2-methyl-spiro-[chroman-4,4'-imidazolidine]-2',5'-dione.

Table 1

| Clinically-tested compound | $ID_{50}$ [M] |
|---|---|
| Compound A | $5.0 \times 10^{-8}$ |
| Compound B | $1.7 \times 10^{-7}$ |
| Compound C | $8.3 \times 10^{-8}$ |

As apparent from the above Experiment, the compounds of this invention have extremely good aldose reductase inhibitory activities.

The compounds provided by the present invention are useful for the treatment and prevention of various chronic symptoms and complications accompanying diabetes which is intractable disease, for example, diabetic cataracts, diabetic neuropathy such as peripheral, neuritis (neuropathy), a variety of arteriosclerotic angiopathy induced by diabetes, diabetic nephritis, microangiopathy such as diabetic retinopathy. The present invention is therefore extremely valuable.

Moreover, the compounds of this invention have extremely low toxicity levels. In this sense, the present invention is also highly valuable because the above-described disease require long-term administration of such drugs in many instances due to the nature of the diseases.

When using the compounds of this invention as compositions for the treatment and prevention of various chronic symptoms and complications accompanying diabetes, they may be administered either orally or parenterally (intramuscular administration, subcutaneous administration, intravenous administration). Their dose levels may vary depending on the type and condition of each disease, the age of each patient. No particular limitation is thus imposed on their dose levels. However, they may usually be

administered at a dosage of about 1—100 mg or preferably about 2—20 mg per day to each adult patient.

In order to formulate the compounds of this invention into dosable forms, they may be prepared into various dosable forms, such as tablets, granules, powders, capsules, injections, suppositories, eye drops and the like, by methods known *per se* in the technical field of drug formulation.

Namely, when preparing orally-dosable solid formulations, each active compound of this invention is added with an excipient and optionally with further additives such as binder, disintegrator, lubricant, colorant, corrigent, followed by formulation of the resulting mixture into tablets, coated tablets, granules, powder or capsules in a usual manner.

Illustrative of such an excipient may include lactose, corn starch, saccharose, glucose, sorbitol, crystalline cellulose. As exemplary binders, may be mentioned polyvinyl alcohol, polyvinyl ether, ethylcellulose, methylcellulose, gum arabic, tragacanth gum, gelatin, shellac, hydroxypropyl starch, polyvinyl pyrrolidone. As exemplary disintegrators, may be mentioned starch, agar-agar, gelatin powder, cyrstalline cellulose, calcium carbonate, sodium hydrogencarbonate, calcium succinate, dextrin, pectin. Illustrative lubricants may include magnesium stearate, talc, polyethylene glycol, silica, hydrogenated vegetable oils. Any colorants may be used so long as their incorporation in drugs has been permitted. As the corrigent, cocoa powder, menthol, and aromatic acid, peppermint oil, borneol, cinnamon powder or the like may be employed. Needless to say, these tablets and granules may be suitably coated if necessary, for example, sugar-coated or gelatin-coated without encountering any problem or inconvenience.

When an injection is desired, each active compound of this invention is added with a pH modifier, buffer, stabilizer, preservative as needed, followed by formulation of the resulting mixture into a subcutaneous, intramuscular or intravenous injection in a usual manner.

Certain Examples of the present invention will hereinafter be described.

## Example 1

6-Fluoro-8-nitro-2-methyl-spiro-[chroman-4,4′-imidazolidine]-2′,5′-dione

(1) Synthesis of 6-fluoro-2-methyl-4-chromanone:

Para-fluorophenol (11.2 g; 0.1 mole) and crotonic acid (17.2 g; 0.2 mole) were dissolved in polyphosphoric acid (100 ml), followed by their reaction at 120°C for 8 hours with vigorous stirring. After cooling the reaction mixture, it was poured in iced 2N-sodium hydroxide solution (450 ml). The resulting mixture was then extracted with chloroform (500 ml). The chloroform layer was washed with 2N-sodium hydroxide solution and then with water, followed by its drying over magnesium sulfate. The solvent was then distilled off. The residue was recrystallized from n-hexane in an amount about 10 times that of the residue to obtain the desired compound, 6-fluoro-2-methyl-4-chromanone [yield: 5.8 g (32%)]. Melting point: 68—69°C.

(2) Synthesis of 6-fluoro-2-methyl-spiro-[chroman-4,4′-imidazolidine]-2′,5′-dione:

Charged in a 300-ml autoclave were the 6-fluoro-2-methyl-4-chromanone (10.8 g; 0.06 mole) obtained in the above procedure (1), acetamide (120 g), potassium cyanate (11.7 g; 0.18 mole) and ammonium carbonate (37.4 g; 0.39 mole). The contents were heated and reacted at 70°C for 24 hours. After completion of the reaction, the reaction mixture was dissolved in water (600 ml) and the resulting aqueous solution was rendered acidic with hydrochloric acid. After collecting the precipitated crystals by filtration, the crystals were dissolved in aqueous 2N-sodium hydroxide solution, followed by an addition of activated carbon. After separating the activated carbon by filtration, the filtrate was rendered acidic with hydrochloric acid and the precipitated crystals were collected by filtration. After washing them with water, they were dried and then recrystallized from ethanol to obtain the desired compound, 6-fluoro-2-methyl-spiro[chroman-4,4′-imidazolidine]-2′,5′-dione [yield: 5.8 g (39%)], the melting point and elementary analysis of which are as follows:

Melting point: 233—235°C.

Elementary analysis for $C_{12}H_{11}FN_2O_3$:

| | C | H | N |
|---|---|---|---|
| Calculated (%) | 57.60 | 4.43 | 11.20 |
| Found (%) | 57.63 | 4.44 | 11.21 |

(3) Synthesis of 6-fluoro-8-nitro-2-methyl-spiro[chroman-4,4′-imidazolidine]-2′,5′-dione:

6-Fluoro-2-methyl-spiro-[chroman-4,4′-imidazolidine]-2′,5′-dione (15 g) obtained in the above procedure (2) was dissolved in acetic acid (160 ml), followed by an addition of conc. nitric acid (20 ml). The resulting mixture was stirred and reacted at 80—110°C for 5 hours. After completion of the reaction, the reaction mixture was poured into water (1.2 l) and the thus-obtained crystalline precipitate was collected by filtration. It was recrystallized from ethanol-water to obtain the desired compound [yield: 11.7 g (66.1%)].

Melting point: 246—248°C.

Elementary analysis for $C_{12}H_{10}FN_3O_5$:

| | C | H | N |
|---|---|---|---|
| Calculated (%) | 48.81 | 3.41 | 14.23 |
| Found (%) | 48.83 | 3.41 | 14.22 |

Example 2

6-Fluoro-8-amino-2-methyl-spiro-[chroman-4,4'-imidazolidine]-2',5'-dione

In water (450 ml), the 6-fluoro-8-nitro-2-methyl-spiro-[chroman-4,4'-imidazolidine]-2',5'-dione obtained in Example 1 was added with reduced iron (16 g) and conc. hydrochloric acid (8.2 ml). They were reacted at 100°C for 2 hours. After completion of the reaction, the reaction mixture was neutralized with sodium carbonate, followed by extraction with ethyl acetate. The ethyl acetate layer was washed, dried and then recrystallized from methanol to obtain the desired compound [yield: 6.0 g (47.7%)].

Melting point: 294—295°C.

Elementary analysis for $C_{12}H_{12}FN_3O_3$:

| | C | H | N |
|---|---|---|---|
| Calculated (%) | 54.33 | 4.56 | 15.84 |
| Found (%) | 54.33 | 4.55 | 15.87 |

Example 3

6-Chloro-8-nitro-2-methyl-spiro-[chroman-4,4'-imidazolidine]-2',5'-dione

(1) Synthesis of 6-chloro-2-methyl-4-chromanone:

Following the procedure (1) of Example 1, the desired compound, 6-chloro-2-methyl-4-chromanone was obtained [yield: 5.8 g (30.0%)]. Melting point: 100—102°C.

(2) Synthesis of 6-chloro-2-methyl-spiro-[chroman-4,4'-imidazolidine]-2',5'-dione:

Repeating the procedure (2) of Example 2 except for the use of the 6-chloro-2-methyl-4-chromanone obtained in the above procedure (1), the desired compound, 6-chloro-2-methyl-spiro-[chroman-4,4'-imidazolidine]-2',5'-dione having the following melting point and elementary analysis was obtained [yield: 8.8 g (55%)].

Melting point: 283—285°C.

Elementary analysis for $C_{12}H_{11}ClN_2O_3$:

| | C | H | N |
|---|---|---|---|
| Calculated (%) | 54.05 | 4.16 | 10.50 |
| Found (%) | 54.10 | 3.71 | 10.55 |

(3) Synthesis of 6-chloro-8-nitro-2-methyl-spiro-[chroman-4,4'-imidazolidine]-2',5'-dione:

Using the 6-chloro-2-methyl-spiro-[chroman-4,4'-imidazolidine]-2',5'-dione obtained in the above procedure (2) as a starting material, the procedure (3) of Example 1 was repeated to obtain the desired compound having the following properties:

Melting point: 279—281°C.

Elementary analysis for $C_{12}H_{10}ClN_3O_5$:

| | C | H | N |
|---|---|---|---|
| Calculated (%) | 46.23 | 3.23 | 13.48 |
| Found (%) | 46.20 | 3.25 | 13.48 |

Example 4

6-Chloro-8-amino-2-methyl-spiro-[chroman-4,4'-imidazolidine]-2',5'-dione

Using the 6-chloro-8-nitro-2-methyl-spiro-[chroman-4,4'-imidazolidine]-2',5'-dione obtained in Example 3 as a starting material, the procedures of Example 2 were repeated to obtain the desired compound having the following properties:

Melting point: above 300°C.

Elementary analysis for $C_{12}H_{12}ClN_3O_3$:

| | C | H | N |
|---|---|---|---|
| Calculated (%) | 51.16 | 4.29 | 14.92 |
| Found (%) | 51.19 | 4.32 | 14.91 |

**Claims for the Contracting States: BE CH DE FR GB IT LI NL SE**

1. A hydantoin derivative having the general formula (I):

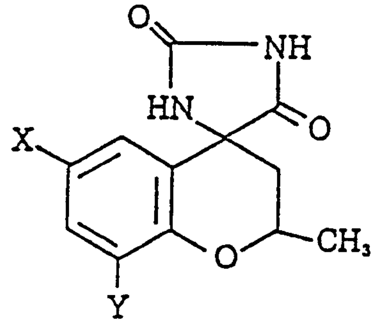

(I)

wherein X represents a halogen atom, and Y represents an amino or nitro group, or a pharmacologically acceptable salt thereof.

2. A compound or a pharmacologically acceptable salt thereof as claimed in claim 1 wherein X is a halolgen atom and Y is an amino group.

3. A compound or a pharmacologically acceptable salt thereof as claimed in claim 1 wherein X is a halogen atom and Y is a nitro group.

4. A compound as defined in claim 1 which is 6-fluoro-8-nitro-2-methyl-spiro-[chroman-4,4'-imidazolidine]-2',5'-dione; or a pharmacologically acceptable salt thereof.

5. A compound as defined in claim 1 which is 6-fluoro-8-amino-2-methyl-spiro-[chroman-4,4'-imidazolidine]-2',5'-dione; or a pharmacologically acceptable salt thereof.

6. A compound as defined in claim 1 which is 6-chloro-8-amino-2-methyl-spiro-[chroman-4,4'-imidazolidine]-2',5'-dione; or a pharmacologically acceptable salt thereof.

7. A compound as defined in claim 1 which is 6-chloro-8-nitro-2-methyl-spiro-[chroman-4,4'-imidazolidine]-2',5'-dione; or a pharmacologically acceptable salt thereof.

8. A process for the preparation of a compound having the general formula:

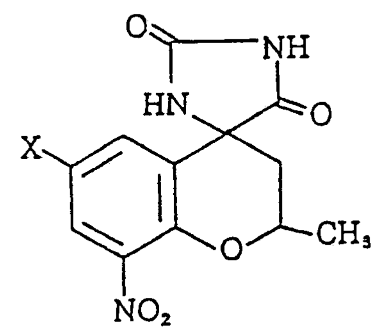

wherein X represents a halogen atom, which comprises nitrating a compound having the general formula:

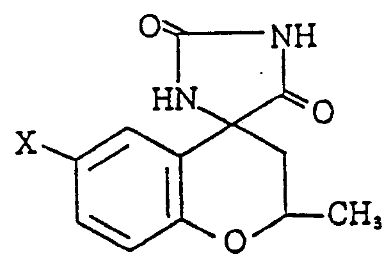

wherein X has the same meaning as defined above.

9. A process for the preparation of a compound having the general formula:

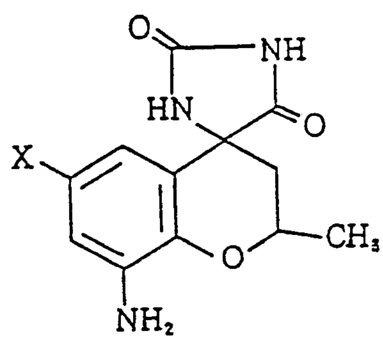

wherein X represents a halogen atom, which comprises reducing a compound having the general formula:

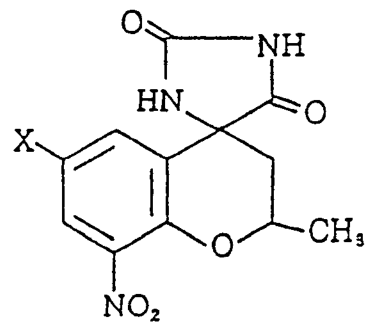

wherein X has the same meaning as defined above.

10. A pharmaceutical composition comprising a therapeutically effective amount of a hydantoin derivative having the general formula I described in claim 1, or a pharmacologically acceptable salt thereof and a pharmaceutical carrier.

11. Use of the compounds of claims 1 to 7 for the preparation of a composition for the treatment and prevention of chronic symptoms and complications accompanying diabetes.

**Claims for the Contracting State: AT**

1. A process for the preparation of a compound having the general formula I

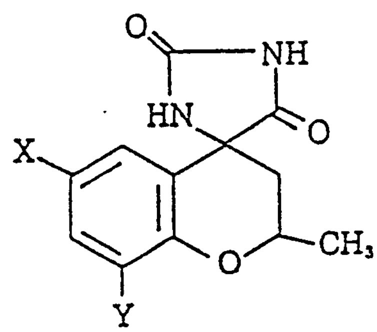

(I)

wherein X represents a halogen atom and Y represents a nitro group, which comprises nitrating a compound having the general formula I wherein X has the same meaning as defined above and Y represents a hydrogen atom.

2. A process for the preparation of a compound having the general formula I wherein X represents a halogen atom and Y represents an amino group comprising reduction of a compound of formula I wherein X represents a halogen atom and Y represents a nitro group.

3. A process for the preparation of a compound of formula I wherein X represents a fluorine atom and Y represents a nitro group comprising nitrating a compound of formula I wherein X represents a fluorine atom and Y represents a hydrogen atom.

4. A process for the preparation of a compound of formula I wherein X represents a fluorine atom and Y represents an amino group which comprises reducing a compound of formula I wherein X represents a fluorine atom and Y represents a nitro group.

5. A process for the preparation of a compound of formula I wherein X represents a chlorine atom and Y represents an amino group which comprises reduction of a compound of formula I wherein X represents a chlorine atom and Y represents a nitro group.

6. A process for the preparation of a compound of formula I wherein X represents a chlorine atom and Y represents a nitro group which comprises nitrating a compound of formula I wherein X represents a chlorine atom and Y represents a hydrogen atom.

7. The use of a compound of formula I wherein X represents a halogen atom and Y represents a nitro group or an amino group for the preparation of a composition for the treatment and prevention of chronic symptoms and complications accompanying diabetes.

**Patentansprüche für die Vertragsstaaten: BE CH DE FR GB IT LI NL SE**

1. Hydantoinderivat der allgemeinen Formel (I)

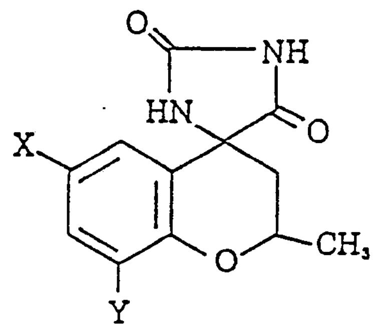

(I)

worin X ein Halogenatom und Y eine Amino- oder Nitrogruppe bezeichnet, oder ein pharmakologisch annehmbares Salz davon.

2. Verbindung oder ein pharmakologisch annehmbares Salz davon gemäss Anspruch 1, worin X ein Halogenatom und Y eine Aminogruppe ist.

3. Verbindung oder ein pharmakologisch annehmbares Salz davon gemäss Anspruch 1, worin X ein Halogenatom und Y eine Nitrogruppe ist.

4. Verbindung gemäss Anspruch 1, nämlich 6-Fluor-8-nitro-2-methyl-spiro-[chroman-4,4'-imidazolidin]-2',5'-dion; oder ein pharmakologisch annehmbares Salz davon.

5. Verbindung gemäss Anspruch 1, nämlich 6-Fluor-8-amino-2-methyl-spiro-[chroman-4,4'-imidazolidin]-2',5'-dion; oder ein pharmakologisch annehmbares Salz davon.

6. Verbindung gemäss Anspruch 1, nämlich 6-Chlor-8-amino-2-methyl-spiro-[chroman-4,4'-imidazolidin]-2',5'-dion; oder ein pharmakologisch annehmbares Salz davon.

7. Verbindung gemäss Anspruch 1, nämlich 6-Chlor-8-nitro-2-methyl-spiro-[chroman-4,4'-imidazolidin]-2',5'-dion; oder ein pharmakologisch annehmbares Salz davon.

8. Verfahren zur Herstellung einer Verbindung der allegemeinen Formel

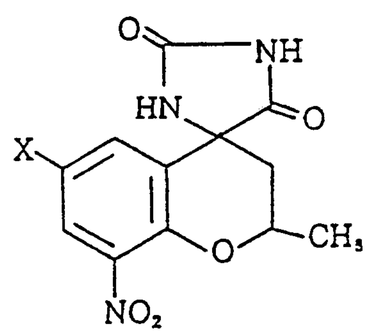

worin X ein Halogenatom bezeichnet, welches das Nitrieren einer Verbindung der allgemeinen Formel

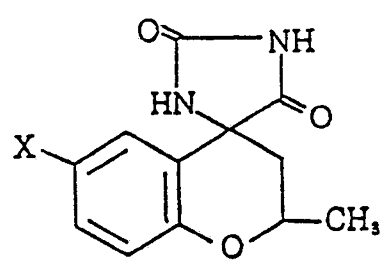

umfasst, worin X die vorerwähnte Bedeutung hat.

9. Verfahren zur Herstellung einer Verbindung der allgemeinen Formel

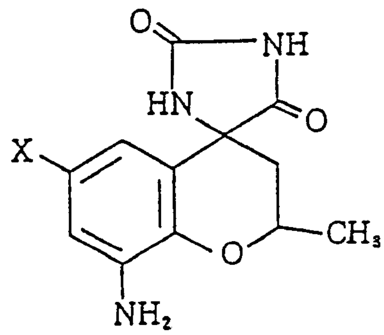

worin X ein Halogenatom bezeichnet, welches das Reduzieren einer Verbindung der allgemeinen Formel

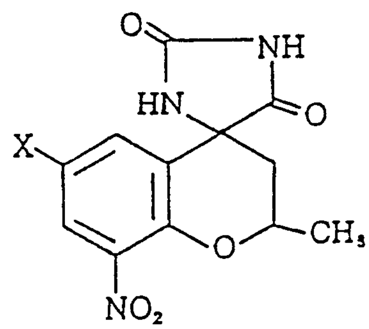

umfasst, worin X die vorerwähnte Bedeutung hat.

10. Pharmazeutische Zusammensetzung, die eine therapeutisch wirksame Menge eines Hydantoinderivates der allgemeinen Formel (I) gemäss Anspruch 1 oder ein pharmakologisch annehmbares Salz davon und einen pharmazeutischen Träger umfasst.

11. Verwendung der Verbindungen gemäss Ansprüchen 1 bis 7 zur Herstellung einer Zusammensetzung zur Behandlung und Prävention von mit Diabetes verbundenen chronischen Symptomen und Komplikationen.

**Patentansprüche für den Vertragsstaat: AT**

1. Verfahren zur Herstellung einer Verbindung der allgemeinen Formel (I)

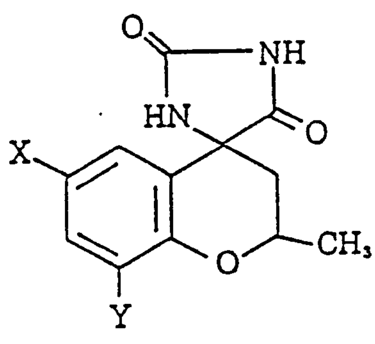

(I)

worin X ein Halogenatom und Y eine Nitrogruppe bezeichnet, welches das Nitrieren einer Verbindung der allgemeinen Formel (I) umfasst, worin X die vorerwähnte Bedeutung hat und Y ein Wasserstoffatom bezeichnet.

2. Verfahren zur Herstellung einer Verbindung der allgemeinen Formel (I), worin X ein Halogenatom und Y eine Aminogruppe bezeichnet, welches das Reduzieren einer Verbindung der Formel (I) umfasst, worin X ein Halogenatom und Y eine Nitrogruppe bezeichnet.

3. Verfahren zur Herstellung einer Verbindung der Formel (I), worin X ein Fluoratom und Y eine Nitrogruppe bezeichnet, das das Nitrieren einer Verbindung der Formel (I) umfasst, worin X ein Fluoratom und Y ein Wasserstoffatom bezeichnet.

4. Verfahren zur Herstellung einer Verbindung der Formel (I), worin X ein Fluoratom und Y eine Aminogruppe bezeichnet, welches das Reduzieren einer Verbindung der Formel (I) umfasst, worin X ein Fluoratom und Y eine Nitrogruppe bezeichnet.

5. Verfahren zur Herstellung einer Verbindung der Formel (I), worin X ein Chloratom und Y eine Aminogruppe bezeichnet, welches das Reduzieren einer Verbindung der Formel (I) umfasst, worin X ein Chloratom und Y eine Nitrogruppe bezeichnet.

6. Verfahren zur Herstellung einer Verbindung der Formel (I), worin X ein Chloratom und Y eine Nitrogruppe bezeichnet, welches das Nitrieren einer Verbindung der Formel (I) umfasst, worin X ein Chloratom und Y ein Wasserstoffatom bezeichnet.

7. Verwendung der Verbindung der Formel (I), worin X ein Halogenatom und Y eine Nitrogruppe oder eine Aminogruppe bezeichnet, zur Herstellung einer Zusammensetzung zur Behandlung und Prävention von mit Diabetes verbundenen chronischen Symptomen und Komplikationen.

**Revendications pour les Etats contractants: BE CH DE FR GB IT LI NL SE**

1. Dérivé de l'hydantoïne de formule générale (I):

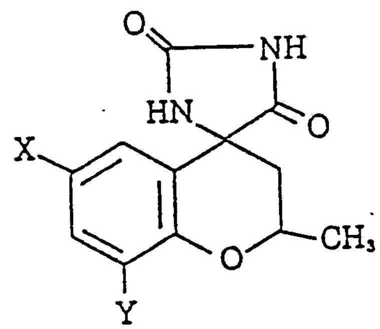

(I)

dans laquelle X représente un atome d'halogène, et Y représente un groupe amino ou nitro, ou un sel pharmacologiquement acceptable de ce composé.

2. Composé ou sel pharmacologiquement acceptable de celui-ci selon la revendication 1 dans lequel X est un atome d'halogène et Y est un groupe amino.

3. Composé ou sel pharmacologiquement acceptable de celui-ci selon la revendication 1 dans lequel X est un atome d'halogène et Y est un groupe nitro.

4. Composé selon la revendication 1 qui est la 6-fluoro-8-nitro-2-méthyl-spiro-[chromane-4,4'-imidazolidine]-2',5'-dione; ou un sel pharmacologiquement acceptable de celui-ci.

5. Composé selon la revendication 1 qui est la 6-fluoro-8-amino-2-méthyl-spiro-[chromane-4,4'-imidazolidine]-2',5'-dione; ou un sel pharmacologiquement acceptable de celui-ci.

6. Composé selon la revendication 1 qui est la 6-chloro-8-amino-2-méthyl-spiro-[chromane-4,4'-imidazolidine]-2',5'-dione; ou un sel pharmacologiquement acceptable de celui-ci.

7. Composé selon la revendication 1 qui est la 6-chloro-8-nitro-2-méthyl-spiro-[chromane-4,4'-imidazolidine]-2',5'-dione; ou un sel pharmacologiquement acceptable de celui-ci.

8. Procédé pour la préparation d'un composé de formule générale:

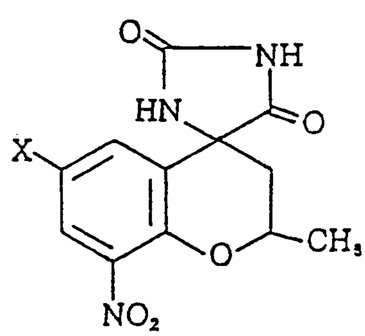

dans laquelle X représente un atome d'halogène, qui comprend la nitration d'un composé de formule générale:

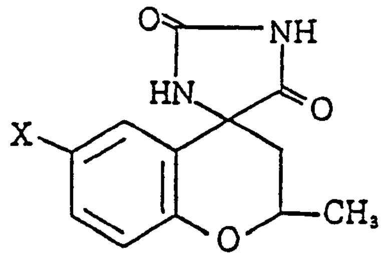

dans laquelle X a la même signification que défini ci-dessus.

9. Procédé pour la préparation d'un composé de formule générale:

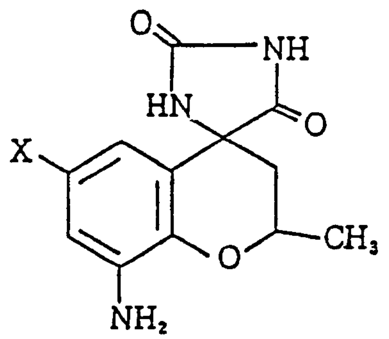

dans laquelle X représente un atome d'halogène, qui comprend la réduction d'un composé de formule générale:

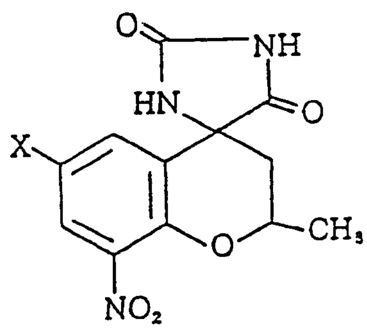

dans laquelle X a la même signification que défini ci-dessus.

10. Composition pharmaceutique comprenant un quantité thérapeutiquement efficace d'un dérivé de l'hydantoïne de formule générale I décrite dans la revendication 1, ou un sel pharmaceutiquement acceptable de celui-ci et un véhicule pharmaceutique.

11. Utilisation des composés des revendications 1 à 7 pour la préparation d'une composition pour le traitement et la prévention des symptômes chroniques et des complications associés au diabète.

**Revendications pour l'Etat contractant: AT**

1. Procédé pour la préparation d'un composé de formule générale:

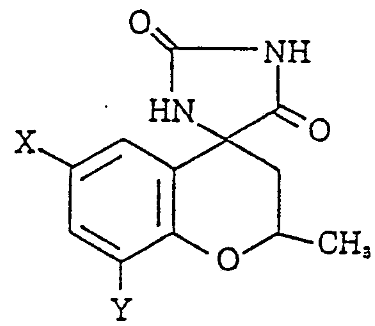

(I)

dans laquelle X représente un atome d'halogène et Y représente un groupe nitro, qui comprend la nitration d'un composé de formule générale I, dans laquelle X a la même signification que défini ci-dessus et Y représente un atome d'hydrogène.

2. Procédé pour la préparation d'un composé de formule générale I dans laquelle X représente un atome d' halogène et Y représente un groupe amino, qui comprend la réduction d'un composé de formule I, dans laquelle X représente un atome d'halogène et Y représente un groupe nitro.

3. Procédé pour la préparation d'un composé de formule I dans laquelle X représente un atome de fluor et Y représente un groupe nitro, comprenant la nitration d'un composé de formule I dans laquelle X représente un atome de fluor et Y représente un atome d'hydrogène.

4. Procédé pour la préparation d'un composé de formule I dans laquelle X représente un atome de fluor et Y représente un groupe amino, comprenant la réduction d'un composé de formule I dans laquelle X représente un atome de fluor et Y représente un groupe nitro.

5. Procédé pour la préparation d'un composé de formule I dans laquelle X représente un atome de chlore et Y représente un groupe amino, comprenant la réduction d'un composé de formule I dans laquelle X représente un atome de chlore et Y représente un groupe nitro.

6. Procédé pour la préparation d'un composé de formule I dans laquelle X représente un atome de chlore et Y représente un groupe nitro, comprenant la nitration d'un composé de formule I dans laquelle X représente un atome de chlore et Y représente un atome d'hydrogène.

7. Utilisation d'un composé de formule I dans laquelle X représente un atome d'halogène et Y représente un groupe nitro ou un groupe amino pour la préparation d'une composition pour le traitement et la prévention des symptômes chroniques et des complications associés au diabète.